Europäisches Patentamt

European Patent Office

Office européen des brevets

(11) Numéro de publication : **0 345 310 B1**

## (12) FASCICULE DE BREVET EUROPEEN

(45) Date de publication du fascicule du brevet :
**12.02.92 Bulletin 92/07**

(21) Numéro de dépôt : **88909508.9**

(22) Date de dépôt : **17.11.88**

(86) Numéro de dépôt international :
**PCT/CH88/00217**

(87) Numéro de publication internationale :
**WO 89/05131 15.06.89 Gazette 89/13**

(51) Int. Cl.⁵ : $A61F\ 9/00$, A61B 17/02

---

(54) **DISPOSITIF ECARTEUR DE TISSU HUMAIN OU ANIMAL.**

---

(30) Priorité : **04.12.87 CH 4735/87**

(43) Date de publication de la demande :
**13.12.89 Bulletin 89/50**

(45) Mention de la délivrance du brevet :
**12.02.92 Bulletin 92/07**

(84) Etats contractants désignés :
**AT BE CH DE FR GB IT LI LU NL SE**

(56) Documents cités :
**EP-A-01 562 218**
**DE-A- 2 951 664**
**DE-A- 3 535 045**
**US-A- 1 706 500**
**US-A- 3 490 455**

(73) Titulaire : **Grounauer, Pierre-Alain**
**38, rue de l'Ale**
**CH-1003 Lausanne, Vaud (CH)**

(72) Inventeur : **Grounauer, Pierre-Alain**
**38, rue de l'Ale**
**CH-1003 Lausanne, Vaud (CH)**

(74) Mandataire : **Ganguillet, Cyril**
**ABREMA Agence Brevets & Marques**
**Ganguillet & Humphrey Rue Centrale 5 C.P.**
**2065**
**CH-1002 Lausanne (CH)**

EP 0 345 310 B1

Jouve, 18, rue Saint-Denis, 75001 PARIS

**Description**

Les dispositifs mécaniques d'écartement des tissus humains ou animaux doivent permettre la vision des éléments anatomiques sous-jacents, mettant ainsi à jour un champ opératoire. Ces dispositifs de présentation des tissus précédemment cachés sont notamment utiles lors d'opérations microchirurgicales, de l'oeil en particulier ou lors d'exploration fonctionnelle de celui-ci. Ils permettent de maintenir écartées les berges d'une incision cutanée, sans autre force que celle délivrée par le dit dispositif. Actuellement on connait de nombreux dispositifs visant à écarter les paupières et à les maintenir éloignées du globe oculaire. En particulier le dispositif décrit dans le brevet US 4.037.589 en donne un exemple. Celui-ci est très rudimentaire et ne comporte qu'une boucle métallique dont les extrêmités concaves permettent d'écarter les paupières. Un autre exemple de dispositif connu est décrit dans le brevet US 1.706.500: ce dispositif est également rudimentaire en ce sens que la cuillère s'adapte mal à la paupière, d'autre part l'agancement coplanaire des bras ne permet pas l'élévation souhaitée lors d'une intervention chirurgicale. Tous ces dispositifs s'inscrivent dans la stratégie de la sécurité opératoire dont l'analyse est la condition du succès chirurgical lui-même. Cette analyse comporte une étude exhaustive de tous les instruments dont va se servir le chirurgien, notamment l'écartement des paupières, geste préliminaire à toute opération oculaire. On trouve décrit en détail tous les éléments fondamentaux de cette chirurgie dans le livre EYE SURGERY, Georg Eisner, Springer Verlag, 1980. Les pages 73 à 78 en particulier renseignent sur les qualités mécaniques particulières que doivent posséder les dispositifs qui se rapportent à la dite description. Il y est dit notamment que le globe oculaire ne doit plus être en contact avec le bord des paupières, ni lécarteur proprement dit. Or il se trouve que les dispositifs d'écartement des paupières à disposition jusqu'ici présentent les nombreux inconvénients tels que cités ci-dessus.

On connaît également divers dispositifs d'écartement destinés aux opérations du corps en général, mais qui ne sont pas adaptés à l'écartement des paupières. En particulier, le brevet américain no 1,706,500 décrit un dispositif écarteur comportant deux bras munis chacun d'une cuillère à l'une de leurs extrémités et reliés à leurs autres extrémités à l'aide d'un ressort destiné à maintenir l'écartement des deux bras. Ce dispositif écarteur est toutefois rudimentaire quant à sa conception et bien entendu inutilisable pour l'écartement des paupières. Le brevet allemand no 2951664 décrit un dispositif de blocage destiné à maintenir un écartement fixé constant entre deux instruments chirurgicaux, gynécologiques ou urologiques. L'une des figures de ce brevet illustre incidemment un écarteur de plaies comportant des extrémités articulées. Cet écarteur comporte deux instruments articulés dans leur partie centrale, à la manière d'une paire de ciseaux. Il est muni du dispositif de blocage qui fait l'objet de ce brevet allemand. Il est absolument exclu d'utiliser un tel instrument pour l'écartement des paupières. Le brevet européen no 0156218 décrit un écarteur d'application manuelle comportant des moyens adhésifs pour l'accrochage au bord d'une ouverture ou d'une incision.

On connait d'autre part un dispositif d'écartement de l'iris, qui est décrit dans le brevet américain no 3,490,455. Il comporte un élément en forme de cuillère à deux courbures destinées à s'adapter à la forme de l'oeil.

Toutefois, comme on l'a déjà dit plus haut, aucun dispositif d'écartement connu, qu'il s'agisse des dispositifs qui viennent d'être mentionnés ou des dispositifs d'écartement de paupières proprement dits, ne permet de réaliser de façon simple l'élévation et l'écartement requis des paupières.

Le but de la présente invention est de proposer un dispositif écarteur pour l'écartement des paupières, qui permette de remédier aux inconvénients mentionnés ci-dessus.

A cet effet, l'invention concerne un dipositif écarteur de tissu humain ou animal, plus particulièrement pour écarter les paupières, selon le préambule de la revendication 1 et tel que revendiqué dans la partie caractérisante de la revendication 1.

On va décrire ci-après, à titre d'exemple, une forme d'exécution du dispositif écarteur de l'invention, en se référant aux dessins annexés sur lesquels:

la figure 1 est une vue en élévation du dispositif écarteur montrant une application lors d'une opération oculaire,

la figure 2 est une vue en détail des cuillères et du boîtier,

la figure 3 est une vue de détail du boîtier et de son axe.

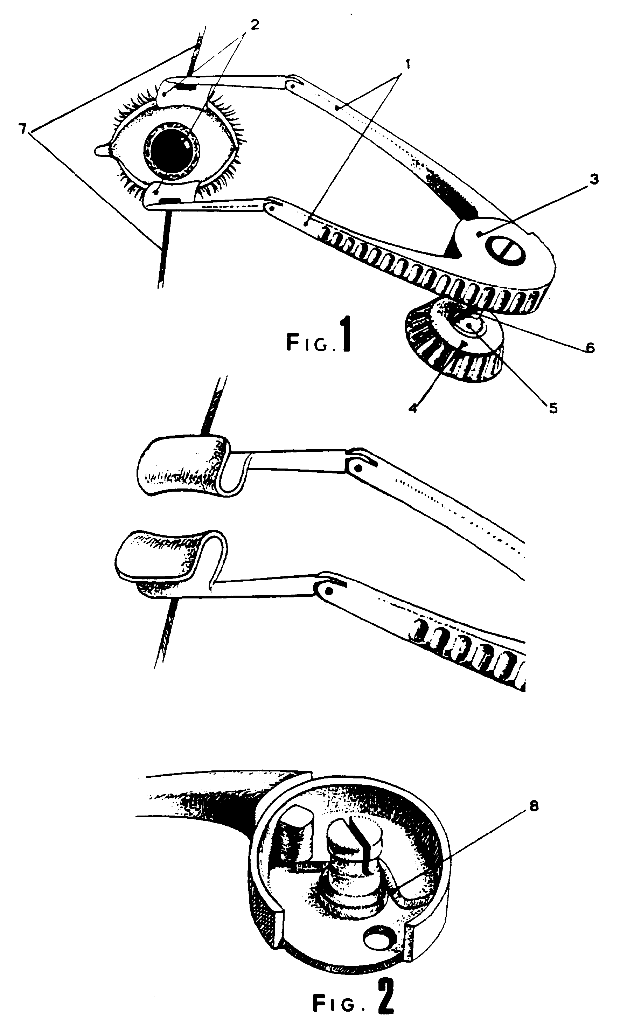

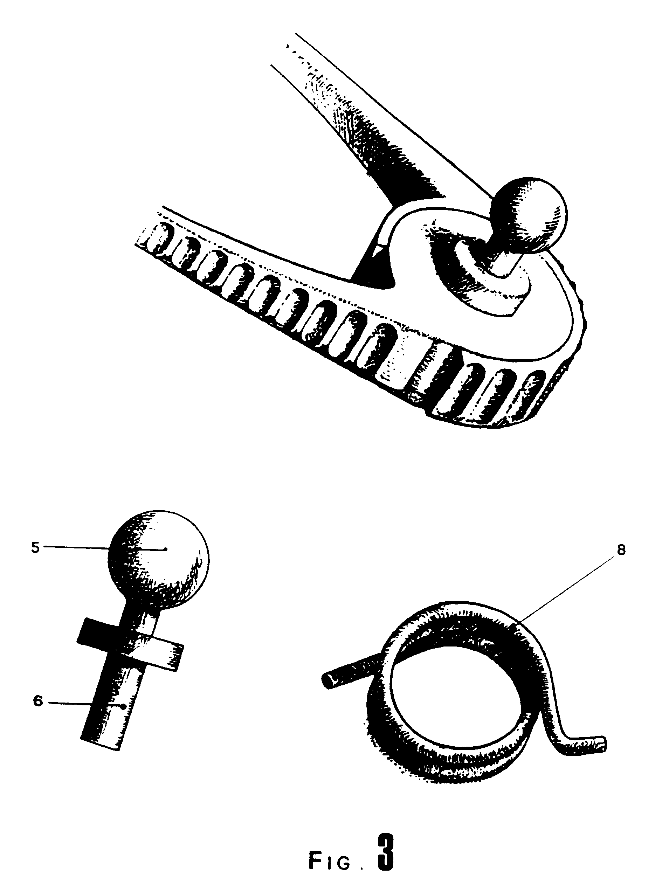

La disposition générale, telle que présentée sur la figure 1 est caractérisée par le fait que les parties distinctes assurant le but recherché sont: les cuillères 2, les rubans 7, les bras 1, les boîtier 3, l'axe 6, la rotule 5, le pied 4. Tous ces éléments sont reliés entre eux de telle sorte qu'une fois collé sur la tempe le dispositif maintienne, par ses bras, les paupières inférieures et supérieures écartées et surélevées, dès lors que leur bord libre est engagé dans la concavité des cuillères 2. D'autre part celles-ci sont mobiles et articulées avec le bout des bras afin de permettre l'élévation souhaitée, action maintenue encore par des rubans 7 prenant appui sur la face extérieure de la cuillère 2 et maintenus sur le champ opératoire par un adhésif. Ce mécanisme assure l'élévation supplémentaire dont le chirurgien pourrait avoir avoir besoin. Le caractère autoporteur de ce dispositif permet son utilisation en salle d'opération ainsi que lors de certaines exploration fonctionnelles de l'oeil. En particulier ce dispositif permet l'écartement et l'élévation des paupières même sur un patient assis.

Le dispositif décrit comporte au bout de chaque bras 1, un boîtier 3 dans lequel se trouve un ressort 8. Celui-ci, mis sous tension, tend automatiquement à écarter les paupières ou les berges de tout autre champ opératoire, sans que le concours d'un deuxième chirurgien soit nécessaire. Le champ opératoire est ainsi dégagé de tout élément diminuant la liberté des gestes chirurgicaux et pouvant rendre plus difficile l'accès aux tissus.

Une fois mis en place le dispositif décrit assure l'élévation des paupières par rapport au globe oculaire grâce à l'articulation existant entre les cuillères 2 et les bras 1. Il se crée ainsi un cercle palpébral, dit de sécurité, éloignant les paupières du globe pour éviter que celles-ci ne provoquent un excès de pression intra-oculaire. Ceci est particulièrement utile en cas d'anesthésie locale de l'oeil, procédé de plus en plus utilisé lors des opérations ambulatoires, de la cataracte en particulier. La possibilité de faire usage de rubans passés à l'intérieur des cuillères augmente encore l'éloignement des paupières. L'usage de bandes autocollantes ou de pinces permet de maintenir l'effet obtenu, tout en assurant l'immobilité du dispositif dont le pied est collé par autocollant double face sur le côté temporal de l'orbite, ce qui lui assure l'immobilité.

En résumé ce dispositif a trois caractéristiques : il est autoporteur, autoécarteur, autoélévateur.

Les cuillères 2 sont adaptées à la forme du globe et des paupières, c'est-à-dire qu'elles possèdent les courbures requises dans le plan anatomique frontal et horizontal. De dimensions variables elles s'adaptent à toutes les grandeurs de globes, du nouveau-né à l'adulte. Elle présentent une surface pleine, contrairement à d'autres dispositifs, afin de cacher les cils qui pourraient entrer en contact avec les instruments ou les fils de suture et favoriser les infections. D'autre part la pression sur les paupières est répartie plus uniformément, évitant ainsi certaines complications décrites avec d'autres dispositifs, à savoir les paralysies post-opératoires de la paupière supérieure. Elles possèdent une fente où peuvent être glissés de fins rubans, qui vont contribuer à l'immobilité dudit dispositif. Elles sont articulées avec les bras pour permettre un réglage de la fonction d'élévation.

Les rubans 7 permettent de régler et de maintenir l'élévation souhaitée, tout en constituant des points de fixation au champ opératoire, concourant ainsi à la stabilité de l'ensemble.

Les bras 1 permettent de transmettre aux cuillères 2 la force libérée par le ressort 8 sous tension inclus dans le boîtier 3. Les bras 1 et les cuillères 2 sont articulées dans un même plan.

Le ressort 8 permet d'assurer la fonction d'autoécartement. A titre d'exemple l'article scientifique paru dans OPHTHAL MIC SURGERY, vol.14, page 575--578,1983,donne des valeurs permettant de déterminer la force du ressort 8.

L'axe 6 et la rotule 5 permettent de transmettre toutes les positions requises pour pouvoir s'adapter aux conditions anatomiques de chaque patient.

Le boîtier 3 peut être séparé de l'axe 6 pour permettre au chirurgien d'exposer une autre partie du globe oculaire.

La face inférieure du pied 4 est recouverte d'un adhésif double face permettant le collage sur la paroi externe correspondant à la tempe du patient. La surface de contact a été choisie pour assurer un maximum d'immobilité.

Ce dispositif peut être fabriqué dans toutes matières à propriétés biocompatibles.

En résumé ce dispositif écarteur offre par rapport aux dispositifs connus les avantages suivants :

1. Immobilité. Celle-ci est assurée par trois points fixes solidaires du champ opératoire et maintenant le cercle palpébral à distance du globe.

2. Adaptabilité. Le dispositif est adaptable aux angles anatomiques par un premier réglage de l'axe de la jambe maintenue dans un pied, puis par un réglage fin grâce à l'articulation des cuillères.

3. Ce dispositif est profilé et conçu de telle sorte qu'aucune vis ou tige ne risque de retenir et de maintenir captifs les fils chirurgicaux lors de leur mise en place.

4. L'écartement des paupières est réalisé par des cuillères à surface pleine, possédant une fente située sur leur face avant à travers laquelle sont passés deux rubans de sécurité.

5. La symétrie du dispositif lui permet d'être posé indifféremment sur l'oeil droit ou sur l'oeil gauche.

6. Le dispositif est conçu de telle sorte qu'il puisse être injecté en matière plastique médicale dont l'avantage est, entre autre la légèreté. De plus il peut être produit par moulage et injection en série, ce qui diminue le coût du dispositif et permet son utilisation en usage unique.

7. Il est non aimantable.

8. Il est radiotransparent.

## Revendications

1. Dispositif écarteur de tissu humain ou animal, plus particulièrement pour écarter les paupières, comportant deux bras (1) ayant chacun une première et une seconde extrémité, la première extrémité de chaque bras étant munie d'une cuillère (2), la seconde extrémité de chaque bras étant reliée à un boîtier (3), l'ensemble étant agencé de façon à maintenir élastiquement l'écartement desdites premières extrémités, caractérisé en ce que les cuillères comportent deux courbures, l'une étant adaptée au cercle palpébral et l'autre étant adaptée à la courbure du globe oculaire, chacune des cuillères étant montée en articulation à l'extrémité du bras correspondant, de façon à pivoter autour d'un axe sensiblement perpendiculaire à l'axe dudit bras.

2. Dispositif selon la revendication 1, caractérisé en ce que le boîtier comporte un ressort (8).

3. Dispositif selon l'une des revendications 1 ou 2, caractérisé en ce que le boîtier comporte un axe (6) articulé relié à un pied (4).

4. Dispositif selon la revendication 3, caractérisé en ce que la face inférieure du pied comporte un adhésif double face.

5. Dispositif selon l'une des revendications précédentes, caractérisé en ce qu'il comporte deux rubans (7), l'une des extrémités de chaque ruban étant respectivement rattachée à l'une des cuillères, les secondes extrémités de chaque ruban étant destinées à constituer des points de fixation permettant de régler et maintenir l'élévation des cuillères.

6. Dispositif selon l'une des revendications précédentes, caractérisé en ce que les deux courbures des cuillères sont constituées par une surface pleine.

7. Dispositif selon l'une des revendications précédentes, caractérisé en ce que l'articulation de chacune des cuillères sur chacun des bras respectifs est disposée à l'extrémité d'une partie allongée en forme de manche.

## Claims

1. Retractor device for human or animal tissue, more particularly for retracting eyelids apart from each other, comprising two arms (1), each arm having a first end and a second end, the first end of each arm being provided with a spoon (2), the second end of each arm being connected to a casing (3), the assembly being arranged to elastically maintain spacing apart of said first ends, characterized in that the spoons comprise two curvatures, one curvature being adapted to the palpebral circle and the other curvature being adapted to the curvature of the eyeball, each of the spoons being articulatingly mounted to the end of the corresponding arm, to pivote about an axis substantially perpendicular to the axis of said arm.

2. Retractor device according to claim 1, characterized in that the casing comprises a spring (8).

3. Retractor device according to one of the claims 1 or 2, characterized in that the casing comprises an articulated shaft (6), connected to a foot (4).

4. Retractor device according to claim 3, characterized in that the lower face of the foot comprises a double face adhesive.

5. Retractor device according to one of the preceding claims, characterized in that it comprises two strips (7), one of the ends of each strip being respectively attached to one of the spoons, the second ends of each strip being intended for providing fixation points making it possible a regulation and maintenance of the raising of the spoons.

6. Retractor device according to one of the preceding claims, characterized in that the two curvatures of the spoons are formed by a solide surface.

7. Retractor device according to one of the preceding claims, characterized in that the articulation of each of the spoons about each of the respected arms is located at the end of an elongated shaft-shaped part.

## Patentansprüche

1. Spreizvorrichtung für menschliches oder tierisches Gewebe, insbesondere zum Spreizen von Augenlidern, mit zwei Armen (1), deren jeder ein erstes und ein zweites Ende aufweist, wobei das erste Ende eines jeden Armes mit einem Greifer (2) versehen ist und das zweite Ende eines jeden Armes mit einem Gehäuse (3) verbunden ist und wobei die gesamte Vorrichtung derart ausgestaltet ist, daß die Spreizung der beiden ersten Enden elastisch beibehalten wird, dadurch gekennzeichnet, daß die Greifer zwei Krümmungen aufweisen, von denen eine an den palpebralen Kreis angepaßt ist und die andere an die Wölbung des Augapfels angepaßt ist, und daß jeder der Greifer gelenkig am Ende des zugehörigen Armes angeordnet ist, dergestalt, daß er um eine Achse verschwenken kann, die im wesentlichen senkrecht zur Achse des Armes verläuft.

2. Vorrichtung nach Anspruch 1, dadurch gekennzeichnet, daß das Gehäuse eine Feder (8) aufweist.

3. Vorrichtung nach einem der Ansprüche 1 oder 2, dadurch gekennzeichnet, daß das Gehäuse eine Achse (6) aufweist, die gelenkig mit einem Fußteil (4) verbunden ist.

4. Vorrichtung nach Anspruch 3, dadurch gekennzeichnet, daß die Unterseite des Fußteils einen Zwei-Seiten-Kleber aufweist.

5. Vorrichtung nach einem der vorhergehenden Ansprüche, dadurch gekennzeichnet, daß sie zwei Bänder (7) aufweist, wobei ein Ende eines jeden Bandes mit jeweils einem der Greifer verbunden ist und das andere Ende als Befestigungspunkt dient, um so eine Anhebung der Greifer einzustellen und festzuhalten.

6. Vorrichtung nach einem der vorhergehenden Ansprüche, dadurch gekennzeichnet, daß die beiden Krümmungen der Greifer je durch eine massive Fläche gebildet werden.

7. Vorrichtung nach einem der vorhergehenden Ansprüche, dadurch gekennzeichnet, daß die Anlenkung eines jeden Greifers am zugehörigen Arm am Ende eines langgestreckten, hülsenförmigen Abschnitts erfolgt.

FIG. 1

FIG. 2

FIG. 3